**Europäisches Patentamt**

**European Patent Office**

**Office européen des brevets**

(19)

(11) Veröffentlichungsnummer : **0 380 045 B1**

(12) # EUROPÄISCHE PATENTSCHRIFT

(45) Veröffentlichungstag der Patentschrift :
**19.08.92 Patentblatt 92/34**

(51) Int. Cl.$^5$ : **A61F 2/34**

(21) Anmeldenummer : **90101279.9**

(22) Anmeldetag : **23.01.90**

(54) **Pfanne für eine Gelenkendoprothese.**

(30) Priorität : **23.01.89 DE 3901885**

(43) Veröffentlichungstag der Anmeldung :
**01.08.90 Patentblatt 90/31**

(45) Bekanntmachung des Hinweises auf die
Patenterteilung :
**19.08.92 Patentblatt 92/34**

(84) Benannte Vertragsstaaten :
**AT CH DE ES FR GB LI**

(56) Entgegenhaltungen :
**EP-A- 0 265 712**
**EP-A- 0 270 744**
**EP-A- 0 277 511**
**EP-A- 0 285 756**

(73) Patentinhaber : **PLUS-Endoprothetik AG**
**Erlenstrasse 4 b**
**CH-6343 Rotkreuz (CH)**

(72) Erfinder : **Sattel, Werner, Prof. Dr. med.**
**Emil-Nolde-Weg 3**
**W-3400 Göttingen (DE)**

(74) Vertreter : **Rehberg, Elmar, Dipl.-Ing.**
**Postfach 3162 Am Kirschberge 22**
**W-3400 Göttingen (DE)**

**Beschreibung**

Die Erfindung bezieht sich auf eine metallene Pfanne für eine Gelenkendoprothese mit den im Oberbegriff des Anspruchs 1 angegebenen Merkmalen. Derartige Pfannen aus Metall oder Metallegierungen werden oft als Bestandteil einer Gelenkendoprothese, insbesondere eines künstlichen Hüftgelenks, benutzt.

Aus der CH-PS 668 180 ist bereits eine metallene Pfanne für eine Gelenkendoprothese mit den im Oberbegriff des Anspruchs 1 angegebenen Merkmalen bekannt, d. h. es findet ein etwa halbkugelförmiger Pfannenkörper Verwendung, der mindestens ein Durchgangsloch mit Ansenkung zwischen Pol und Äquator für den versenkten Einsatz einer Knochenschraube aufweist. Insbesondere ist das Durchgangsloch langlochartig ausgebildet und wird von drei Halbkugelschalen sowie einer wannenförmigen Ausnehmung umschlossen. Innerhalb des halbkugeligen Pfannenkörpers sind drei solcher langgestreckter Durchgangslöcher vorgesehen, die wahlweise an verschiedenen vorgegebenen Stellen dem Einsatz von Knochenschrauben dienen. Der bekannte Pfannenkörper weist auf seiner äußeren konkaven Oberfläche eine glatte Gestaltung auf. Er trägt darüber hinaus in dem Anordnungsschema der langlochartigen Durchgangslöcher noch weitere schlitzartige Durchbrüche in regelmäßiger Verteilung über die Oberfläche. Diese Durchbrüche sollen der Sekundärfixation durch An- und Einwachsen des Knochengewebes dienen. Die Primärfixation wird ausschließlich durch Knochenschrauben erzielt.

Diese bekannte Pfanne weist gravierende Nachteile auf: Während der Operation, bei der sich der Patient in Rücken- oder Seitenlage befindet, ergibt sich eine individuelle Beckenkippung und damit eine individuelle Lage des Beckenknochens. Der einzusetzende Pfannenkörper läßt neben den langlochartigen Durchbrüchen nur erkennen, wo die drei langlochartigen Durchgangslöcher vorgesehen sind, die letztlich von den Knochenschrauben durchdrungen werden sollen. Damit gibt es an dem Pfannenkörper keinen Anhaltspunkt für den Operateur, wie er entsprechend der individuell variierenden Beckenkippung den Pfannenkörper plazieren soll und in welcher Richtung er die Bohrungen für die Knochenschrauben durchführen soll, damit die Schrauben nicht in das kleine Becken oder nach außen aus der Knochenmasse austreten, sondern auf ihrer ganzen Länge innerhalb eines schmalen Bereichs einer Knochenanhäufung des Beckens zu liegen kommen. Der Operateur hat also einerseits keinen Anhaltspunkt, mit welcher Drehung um die Polachse der Pfannenkörper eingesetzt werden soll und er hat zweitens keine Anhaltspunkt dafür, in welcher Richtung die Löcher zum Einsetzen der Knochenschrauben gebohrt werden sollen. Eine Winkelverfehlung birgt die Gefahr es Durchtretens der Schrauben mit erheblichen Nachteilen und Verletzungen des Patienten.

Weiterhin ist nachteilig, daß die Primärfixation allein durch die Knochenschrauben erzielt wird. Aus diesem Grund muß die Anzahl der Knochenschrauben relativ hoch gewählt werden. In dem Ausführungsbeispiel sind vier Knochenschrauben dargestellt, wobei eine davon in einem Bereich außerhalb des eigentlichen halbkugeligen Pfannenkörpers zur Anwendung kommt. Die Knochenschrauben dienen bei diesem Stand der Technik nicht nur allein der Primärfixation, sondern auch einer Verdrehsicherung des Pfannenkörpers in der menschlichen Knochenpfanne.

Der Erfindung liegt die Aufgabe zugrunde, eine Pfanne dieser Art so weiterzubilden, daß dem Operateur durch die Ausbildung der Pfanne eine Hilfe gegeben wird, in welcher Richtung der Pfannenkörper in das Pfannenlager am Beckenknochen so ausgerichtet eingeschlagen werden kann, daß das Durchgangsloch für das Einsetzen einer Knochenschraube so angeordnet ist, daß beim Bohren und beim nachfolgenden Einsetzen der Knochenschraube nicht die Gefahr besteht, daß diese in das kleine Becken oder nach außen durchdringt.

Erfindungsgemäß wird dies in Verbindung mit den im neuen Anspruch 1 definierten kennzeichnenden Merkmalen erreicht. Wichtig ist dabei zunächst, daß der Pfannenkörper auf seiner konvexen Oberflächen entsprechend der Form der Facies lunata angeordnete Erhebungen mit scharfen Kanten aufweist. Hierauf ist es zurückzuführen, daß die Erhebungen, die hier der Primärfixation dienen, nur an den Stellen gemäß der Form der Facies lunata angeordnete Erhebungen tragen, also dort, wo tatsächlich auch Druck übertragen werden kann. Dies wiederum gestattet es, bei der Herrichtung des Pfannenlagers lediglich eine Entknorpelung herbeizuführen. Ein Verlust an Knochensubstanz tritt dabei nicht auf.

Die Erfindung fußt weiterhin auf der Erkenntnis, daß der Incisura acetabuli genau um 180° entgegengesetzt an der natürlichen Pfanne ein stabiler Knochenblock zur Verfügung steht, der zur Aufnahme der relativ langen Knochenschrauben geeignet ist, ohne daß dabei die Gefahr besteht, daß die Knochenschrauben in das kleine Becken oder nach außen durchtreten. Insoweit ist auf der äußeren Oberfläche des Pfannenkörpers eine feldmäßige Kennzeichnung entsprechend der Lage der Incisura acetabuli angeordnet, so daß der Operateur den Pfannenkörper so gedreht in das natürliche Pfannenlager am Beckenknochen einsetzen kann, daß diese feldmäßige Kennzeichnung der Incisura acetabuli zugekehrt angeordnet ist. Das Durchgangsloch ist auf der um 180° versetzt gegenüberliegenden Seite dieser Kennzeichnung angeordnet, also dann genau an der Stelle, an der der beschriebene relativ massive Knochenblock zur Aufnahme der Knochenschrauben beginnt bzw. radial anschließt. Damit liegt der Ansatzpunkt bzw. Beginn der Bohrung fest, nämlich die Stelle, an die nach dem

EP 0 380 045 B1

Einsetzen der Knochenschrauben die Köpfe dieser Knochenschrauben gelangen. Um nun dem Operateur auch noch eine Hilfe zu geben, in welcher Richtung er die Bohrlöcher führen muß, befindet sich auf der inneren Oberfläche der Pfanne eine nach Art eines Längengrads verlaufende Markierung, und zwar genau an der Stelle innen, an der außen die feldmäßige Kennzeichnung angeordnet ist. Durch diese Markierung wird eine Richtung vorgegeben, die parallel zu einer optimalen Bohrrichtung verläuft, so daß der Operateur insoweit einen Anhaltspunkt zum Einhalten einer optimalen Bohrrichtung hat.

Damit ergeben sich noch weitere Vorteile. Die Erhebungen haben bereits Doppelfunktion. Sie dienen der Primärfixierung und der Rotationssicherung. Auch die Knochenschrauben dienen diesen beiden Zwecken, so daß insgesamt der Vorteil erreicht wird, daß das betr. Gelenk nach der Operation und nach der damit eingetretenen Primärfixierung bereits voll belastet werden kann.

Mit dem Einschlagen des Pfannenkörpers bekommt dieser entweder sofort seinen ordnungsgemäß korrekten Sitz im Pfannenlager. Es tritt also bereits eine endgültige Verklemmung ein. Andererseits ist es möglich, den Sitz noch geringfügig zu korrigieren und die Verklemmung im Sinne einer Primärverklemmung anzuwenden.

Der Pfannenkörper weist mindestens ein Durchgangsloch mit Ansenkung für den versenkten Einsatz einer Knochenschraube auf, wobei das Durchgangsloch zwischen Pol und Äquator auf der der Incisura acetabuli gegenüberliegenden Seite angeordnet ist. Im Zusammenhang damit wird der besondere Vorteil des Einschlagen des Pfannenkörpers - also ohne Eindrehvorgang - vollends offenbart. Auf diese Weise ist es möglich, ein Durchgangsloch bei der Herstellung des Pfannenkörpers an der entsprechenden Stelle, wie beschrieben, vorzusehen, so daß dieses Durchgangsloch nach dem Einschlagen des Pfannenkörpers genau an eine solche Stelle im Pfannenlager des Beckenknochens zu liegen kommt, an der sinnvollerweise genügend Knochensubstanz für den Einsatz einer auch vergleichsweise langen Knochenschraube vorhanden ist, und zwar ohne daß die Gefahr besteht, daß das Ende der Knochenschraube in das kleine Becken eindringen würde oder eine sonstige Beeinträchtigung des Patienten nach sich ziehen könnte. Wie ersichtlich, ist die Anordnung eines Durchgangslochs bei einem Pfannenkörper, der durch einen Eindrehvorgang im Pfannenlager fixiert wird, nicht möglich, weil es sich nicht abschätzen läßt, auf welchem Verdrehwinkel der Eindrehvorgang beendet wird. Die Lage des Durchgangslochs wäre aber dann von dem Ende des Drehwinkels abhängig. Diese Abhängigkeit besteht bei dem erfindungsgemäß ausgebildeten Pfannenkörper nicht mehr. Gleichzeitig wird durch die Lage des Durchgangslochs etwa auf halbem Weg zwischen Pol und Äquator dem Operateur angezeigt, in welcher Relativlage zu der Incisura acetabuli er den Pfannenkörper in das Pfannenlager einzusetzen hat, nämlich so, daß der dem Durchgangsloch gegenüberliegende Bereich deckungsgleich zu der Incisura acetabuli zu liegen kommt. Hierdurch wird es wiederum möglich, den Pfannenkörper nicht rotationssymmetrisch auszubilden, sondern jeden Bereich vorteilhaft beanspruchungsgerecht und nach der gewünschten Funktion auszugestalten. Dies zielt auch darauf ab, dem schalenartigen Pfannenkörper über den Umfang gesehen eine unterschiedliche Wandstärke zu geben, derart, daß im Bereich des Durchgangslochs die größte Wandstärke vorhanden ist, um den Schraubenkopf vollständig versenken zu können, während auf der gegenüberliegenden Seite im Bereich der Incisura acetabuli, also dort, wo kaum oder nur sehr geringe Kräfte überhaupt auftreten, die Wandstärke des Pfannenkörpers entsprechend gering gehalten werden kann. Die Erhebungen mit ihren scharfen Kanten können im wesentlichen in Richtung des Verlaufs von Längen- und Breitengraden an der Kugel angeordnet sein. Diese Erhebungen entsprechend dem Verlauf von Breitengraden erstrecken sich nur über einen vergleichsweise kurzen Bereich, während die in Richtung des Verlaufs von Längengraden auf einer Kugel angeordneten Erhebungen durchaus vergleichsweise länger ausgebildet sein können. Die letzteren Erhebungen können sogar über weite Bereiche der Oberfläche der Halbkugel durchgehend vorgesehen sein. Die scharfen Kanten dieser ersten Art von Erhebungen dienen dazu, in Einschlagrichtung des Pfannenkörpers in die Knochensubstanz einzuschneiden und dabei eine Verankerung herbeizuführen, so daß der Pfannenkörper zugleich gegen Rotation gesichert im Pfannenlager aufgenommen ist. Die zusätzlichen Erhebungen, die dazu etwa rechtwinklig angeordnet sein können, dienen dazu, ein Herausfallen des Pfannenkörpers aus dem Pfannenlager zu verhindern und Angriffspunkte für ein Knochenwachstum zu bilden, so daß auf diese Weise Verbindungspunkte und Stellen zwischen Pfannenkörper und Pfannenlager gebildet werden. Die beiden Arten von Erhebungen können direkt ineinander übergehen. Es versteht sich, daß diese Erhebung je nach ihrer Entfernung vom Pol mit verschiedener Höhe vorgesehen sein können, wobei die Höhe in Richtung von Pol auf Äquator wachsend ausgebildet ist.

An sich können die Erhebungen auf der konvexen Oberfläche über die gesamte Oberfläche entsprechend der Facies lunata verteilt angeordnet sein, wobei ihre Anordnung nur entsprechend der Einschlagrichtung ausgerichtet sein muß. Wichtig ist es, daß die Erhebungen entsprechend der Form der Facies lunata der natürlichen Pfanne im Beckenknochen angeordnet sind. Dabei handelt es sich um eine mond- oder sichelförmige flächige Anordnung, die auf der einen Seite und im mittleren Bereich offen ist. In diesen offenen Bereichen ist es nicht erforderlich, Erhebungen anzuordnen, da hier eine tragende Verbindung zu der knöchernen Substanz

3

des Beckenknochens ohnehin nicht eintritt.

Die Erhebungen können zumindest teilweise U-förmige Gestalt aufweisen, wobei die Öffnung des U dem Pol der Pfanne zugekehrt angeordnet ist. Damit weist jede Erhebung in Form der Schenkel des U zwei Schneiden auf, die sich in Richtung des Verlaufs von Längengraden auf einer Kugel erstrecken, während der Grund des U dabei als die zusätzlichen Erhebungen fungiert. Diese Form stellt eine besonders vorteilhafte Ausführungsform der Erhebungen dar. Die polnahen Erhebungen können auch ringförmige Gestalt besitzen.

Das Durchgangsloch kann als Langloch ausgebildet und in Richtung des Verlaufs des Längengrades angeordnet sein, so daß sich dieses in einem gewissen Winkelbereich vom Pol hin auf den Äquator erstreckt. Damit ist die Möglichkeit gegeben, im Bereich dieses Langlochs mit größerer Variabilität ein oder mehrere Knochenschrauben zur Anwendung zu bringen, um auf diese Weise eine Sekundärverklemmung des Pfannenkörpers im Pfannenlager zu erzielen. Mit einer solchen Sekundärverklemmung wird wiederum der besondere Vorteil erreicht, daß die Gelenkendoprothese von Anfang an mit dem vollen Körpergewicht belastet werden kann und dabei nicht die Gefahr besteht, daß sich der Pfannenkörper im Pfannenlager des Beckenknochens noch nennenswert verlagert. Es versteht sich, daß das oder die Durchgangslöcher mit den entsprechenden Ansenkungen für die Schraubenköpfe der Knochenschrauben versehen sind. Durch die Gestaltung der Auflagefläche des Schraubenkopfs in Verbindung mit der Gestaltung der Fläche der Ansenkung kann dem Operateur ein Winkelbereich vorgegeben werden, innerhalb dessen er die Knochenschrauben anzuwenden hat. Bevor die Knochenschrauben eingedreht werden und damit die Sekundärverklemmung eintritt, müssen entsprechende Sacklöcher in dem Beckenknochen gebohrt werden, in die die Knochenschrauben dann eingeschraubt werden. Es ist durchaus möglich, diese Sacklöcher bewußt etwas exzentrisch zu der Achse der Durchgangslöcher, beispielsweise mit Hilfe exzentrischer Bohrbüchsen, zu bohren, damit beim Anziehen der Knochenschraube letztendlich der Pfannenkörper noch eine geringfügige versetzende Bewegung relativ zum Pfannenlager ausführt. Dadurch graben sich die Erhebungen mit ihren scharfen Kanten nur noch besser in die Knochensubstanz des Beckenknochens ein und der Sitz wird noch fester und sicherer. Weiterhin eröffnet sich die Möglichkeit, mehr als eine Schraube in diesem Langloch zum Einsatz zu bringen. Auch das Langloch zeigt dem Operateur verläßlich die Stelle, an der sich der Teil des Pfannenkörpers befindet, der der Incisura actabuli abgekehrt angeordnet werden muß.

Zusätzlich zu dem als Langloch ausgebildeten Durchgangsloch können zwei weitere Durchgangslöcher in symmetrischer Anordnung zu der von dem Langloch aufgespannten Ebene vorgesehen sein. Damit wird die Anzahl der einzusetzenden Knochenschrauben erhöht und die Befestigung wird noch sicherer. Gleichzeitig ist es damit möglich, die Pfanne sowohl für das linke wie auch für das rechte Gelenk bei einem Patienten zu benutzen. Auch diese Durchgangslöcher sind selbstverständlich mit Ansenkung versehen, wobei die Fläche dieser Ansenkung insbesondere kegelförmig gestaltet sein kann und in Verbindung mit einer kugelförmigen Anlagefläche am Schraubenkopf der Knochenschraube zusammenwirkt.

Es ist möglich, daß die innere Oberfläche des Pfannenkörpers gegenüber der äußeren Oberfläche des Pfannenkörpers exzentrisch angeordnet ist, wobei die Lage der Exzentrizität so gewählt ist, daß im Bereich der Durchgangslöcher eine große und im Bereich der Incisura acetabuli eine geringe Wandstärke entsteht. Damit wird die Wandstärke gleichzeitig an der Stelle besonders groß, an der einerseits die versenkten Köpfe der Knochenschrauben zu liegen kommen und an der andererseits die größte Belastung, insbesondere beim Stehen, eintritt. Diese Ausbildung ist insbesondere für kleine Pfannen wichtig, bei denen der Durchmesser der äußeren Oberfläche des Pfannenkörpers entsprechend klein ist. Trotzdem sollte die innere Oberfläche des Pfannenkörpers möglichst groß sein bzw. der zugehörige Radius möglichst groß gewählt sein, damit das in jedem Fall in den Pfannenkörper einzufügende Einsatzstück aus Kunststoff mit einer ausreichenden Wandstärke bemessen werden kann.

Der halbkugelförmige Pfannenkörper kann im Bereich seines Pols gegenüber der Kreis- bzw. Kugelform mit einer Abflachung versehen sein. Dies erbringt eine bessere Anpassung an das natürliche Pfannenlager.

Die Erfindung wird anhand verschiedener Ausführungsformen weiter erläutert und beschrieben. Es zeigen:

Figur 1 eine Seitenansicht der Pfanne in einer ersten Ausführungsform,

Figur 2 eine Draufsicht auf die Pfanne in einer zweiten Ausführungsform,

Figur 3 einen Schnitt gemäß der Linie III-III in Figur 2,

Figur 4 einen Schnitt gemäß der Linie IV-IV in Figur 2,

Figur 5 einen Schnitt gemäß der Linie V-V in Figur 2 und

Figur 6 eine teilweise geschnittene Seitenansicht der Pfanne in einer weiteren Ausführungsform.

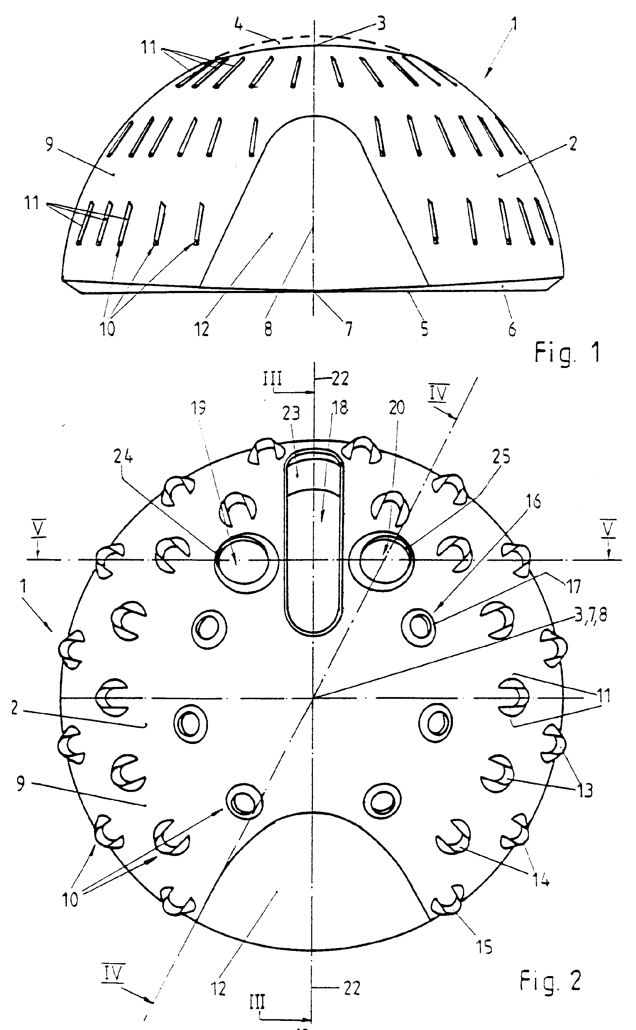

Die Figur 1 zeigt eine Pfanne 1 mit einer äußeren konvexen Oberfläche 2, die etwa die Form einer Halbkugel besitzt. Diese Oberfläche 2 ist im Bereich eines Pols 3 gegenüber der Kreis- bzw. Kugelform mit einer Abflachung 4 versehen. Im Bereich eines Äquators 5, der auch nach Art eines Breitengrads verlaufen kann, besitzt die Pfanne 1 einen Rand 6 mit der dargestellten Formgebung. Die konvexe Oberfläche 2 der Kugel besitzt ein Zentrum 7. Eine Polachse (8) erstreckt sich durch den Pol 3 und das Zentrum 7 hindurch. Die Pfanne 1 weist einen Pfannenkörper 9 auf, der eine schalenartige Gestalt besitzt, wie später noch erläutert wird. Auf der äußeren konvexen Oberfläche 2 sind Erhebungen 10 mit scharfen Kanten 11 vorgesehen. Die scharfen Kanten 11 der Erhebungen 10 sind so angeordnet, daß sie sich in Richtung des Verlaufs von Längengraden an einer Kugel auf der Oberfläche 2 des Pfannenkörpers 9 erstrecken. Die Erhebungen 10 können, müssen jedoch nicht durchgehend vorgesehen sein, sondern können in einer Anordnung, wie dargestellt, auch mit relativ großen Unterbrechungen ausgebildet sein. Die in Figur 1 dargestellten Erhebungen 10 mit ihren Kanten 11 erstrecken sich geradlinig auf der Oberfläche 2 des Pfannenkörpers 9. An einer bestimmten Stelle des Umfangs des Pfannenkörpers 9 besitzt die Pfanne 1 auf ihrer Oberfläche 2 eine feldmäßige Kennzeichnung 12, die in vielerlei Weise ausgebildet sein kann, beispielsweise durch eine Oberflächenpolitur, einen Chromauftrag o. dgl. Wichtig ist nur, daß die Kennzeichnung 12 das Erkennen dieser Stelle gegenüber dem übrigen Umfang der Oberfläche 2 des Pfannenkörpers 9 gestattet. Das Feld der Kennzeichnung 12 ist hier frei von Erhebungen 10. Sofern in diesem Feld trotzdem Erhebungen 10 vorgesehen sind, stören diese nicht, nützen jedoch auch nichts. Die Kennzeichnung 12 kann mit der Stelle am Umfang des Pfannenkörpers 9 zusammenfallen, der die geringste Wandstärke aufweist.

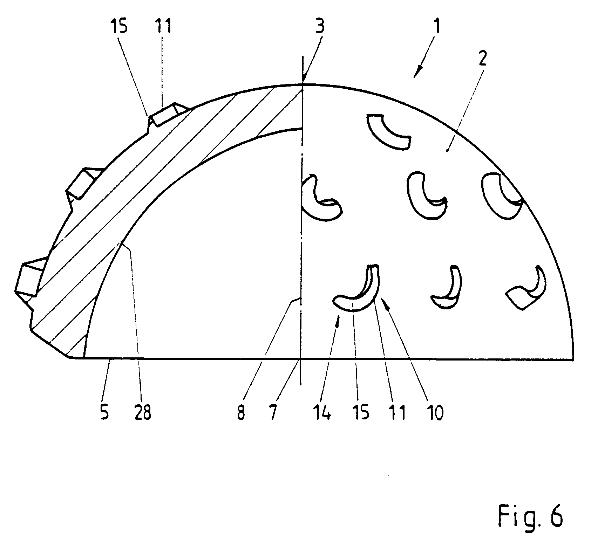

Figur 2 zeigt eine Draufsicht auf eine Pfanne 1 in einer zweiten Ausführungsform, und zwar auf die konvexe Oberfläche 2 des Pfannenkörpers 9. Die auf der Oberfläche 2 angeordneten Erhebungen 10 sind hier etwas anders ausgebildet. Man erkennt, ausgehend vom Äquator 5, zwei Reihen von Erhebungen 10, die als düsenförmige Ausstülpungen 13 bezeichnet werden können. Diese Ausstülpungen 13 besitzen etwa die Gestalt eines U, wobei dessen beiden Schenkel in Richtung des Verlaufs der Längengrade an der Kugel ausgebildet und angeordnet sind. Diese beiden Schenkel tragen dann auch die sich in Richtung der Längengrade erstreckenden scharfen Kanten 11. Vereint mit diesen Kanten 11 bildet der Grund des U jeweils zusätzliche Erhebungen 14 mit scharfen Kanten 15, die sich hier kreisbogenförmig und damit im wesentlichen quer zur Richtung der scharfen Kanten 11 erstrecken. Die Kanten 15 sind damit so ausgerichtet und angeordnet, daß sie in Richtung von Breitengraden auf einer Kugel vorgesehen sind. Man kann die Erhebungen 10, 14 auch als kraterartige Ausstülpungen 13 bezeichnen, die in Richtung auf den Pol 3 offen gestaltet sind. Außer diesen zwei Reihen von Ausstülpungen 13 ist eine weitere Reihe von Erhebungen 10, 14 vorgesehen, die dem Pol 3 am nächsten angeordnet ist. Diese können als düsenartige Ringe 16 mit umlaufenden scharfen Kanten 17 bezeichnet werden. Diese Ringe 16 besitzen grundsätzlich eine ähnliche Gestalt wie auch die Ausstülpungen 13. Sie sind lediglich in Richtung auf den Pol 3 nicht offen ausgebildet, so daß an ihnen die querstehenden zusätzlichen Erhebungen 14 gleichsam zweimal auftreten. Aus der Darstellung gemäß Figur 2 ist auch erkennbar, daß der radiale Überstand der Erhebungen 10, 14 vom Pol 3 in Richtung auf den Äquator 5 zunehmend ausgebildet ist, wie es im übrigen noch deutlicher aus Figur 6 hervorgeht. Die Oberfläche 2 um die Erhebungen 10, 14 herum kann rauh ausgebildet sein, um die Verbindung zu wachsender Knochensubstanz zu verbessern.

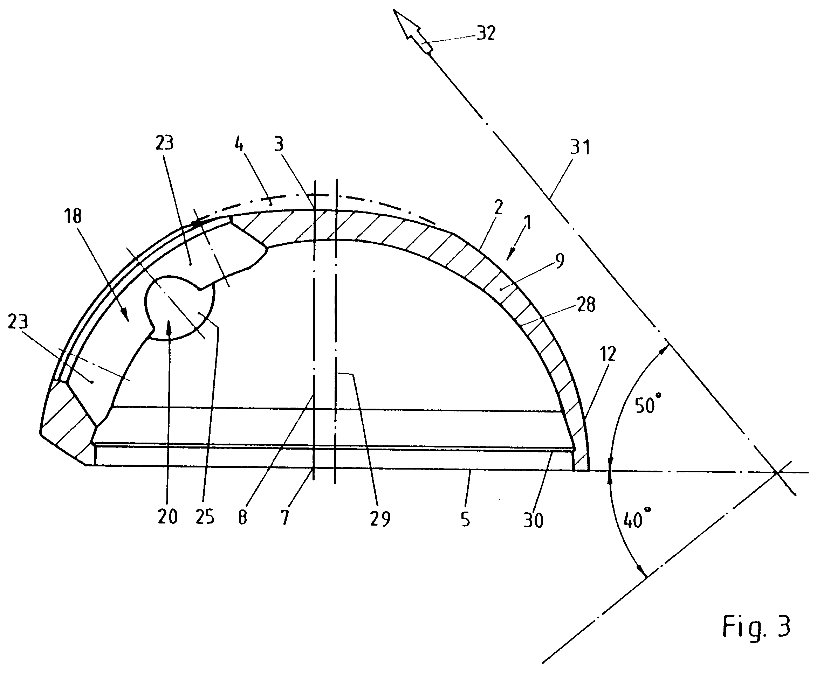

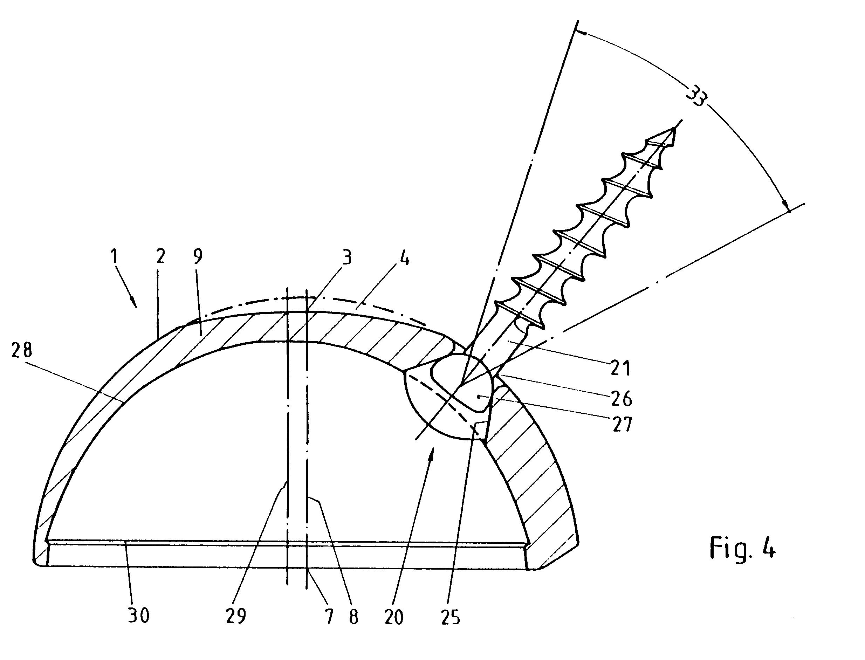

Aus Figur 2 ist auch erkennbar, daß der Pfannenkörper 9 auf der der feldmäßigen Kennzeichnung 12 gegenüberliegenden Seite Durchgangslöcher 18, 19, 20 aufweist, die dem Einsatz einer oder mehrere Knochenschrauben 21 dienen, wie dies anhand von Figur 4 ersichtlich ist. Das Durchgangsloch 18 kann dabei als Langloch ausgebildet sein. Es erstreckt sich vorzugsweise ebenfalls in Richtung eines Längengrads. Die beiden Durchgangslöcher 19 und 20 ergänzen einander bzw. sind symmetrisch zu einer Ebene 22 gemäß der Schnittangabe III-III angeordnet, so daß die Durchgangslöcher 19 und 20 bei Verwendung der Pfanne 1 im Bereich des linken bzw. rechten Hüftgelenks des Menschen ihre Funktion tauschen. Es wäre natürlich auch möglich, die beiden Durchgangslöcher 19 und 20 durch ein Langloch in Richtung des Verlaufs eines Breitengrads miteinander zu verbinden und das Durchgangsloch 18 in zwei Durchgangslöcher aufzulösen. Es kann auch eine andere Konfiguration von Durchgangslöchern gewählt werden. Wichtig ist nur, daß diese Durchgangslöcher 18, 19, 20 in dem Bereich gegenüberliegend zu der Kennzeichnung 12 vorgesehen sind und für die Links-Rechts-Anwendung symmetrisch zur Ebene 22 verteilt angeordnet sind. Weiterhin ist wichtig, daß jedes dieser Durchgangslöcher 18, 19, 20 mit einer insbesondere kegelförmigen Ansenkung 23, 24, 25 versehen ist, die mit einer insbesondere kugelförmigen Auflagefläche 26 am Kopf 27 der Knochenschraube 21 zusammenarbeitet. Auf die Darstellungen der Figuren 3 und 4 wird besonders hingewiesen. Außerdem muß der schalenartige Pfannenkörper 9 in dem Bereich, in welchem die Durchgangslöcher 18, 19, 20 bzw. die Köpfe 27 der Knochenschrauben 21 angeordnet werden, eine solche Wandstärke aufweisen, daß die Köpfe 27 nach dem Einschrauben der Knochenschrauben 21 völlig versenkt sind.

Die Figuren 3 und 4 zeigen, daß der Pfannenkörper 9 neben seiner äußeren konvexen Oberfläche 2 auch eine innere konkave Oberfläche 28 aufweist, die ebenfalls kugelförmig ausgebildet ist, wobei das Zentrum dieser Kugel auf einer Achse 29 liegt, die mit Abstand zu der Polachse 8 der konvexen Oberfläche 2 angeordnet ist, so daß, wie ersichtlich, die Ausbildung des Pfannenkörpers 9 mit seinen beiden Oberflächen 2 und 28 zwar symmetrisch zu der Ebene 22 vorgenommen ist, jedoch die Wandstärke im Bereich der Kennzeichnung 12 am geringsten und im Bereich der Durchgangslöcher 18, 19, 20 am größten ist. Diese exzentrische Ausbildung ist besonders wichtig für kleine Größen des Pfannenkörpers 9 bzw. der Pfanne 1. Die Pfannen 1 werden in individuellen Größen von 44 bis 64 mm (Durchmesser der Kugel der äußeren Oberfläche 2) in Zwei-Millimeter-Abstufungen hergestellt, um die jeweilige Pfanne 1 individuell für den Patienten auswählen zu können, und zwar nach der Größe seines zu ersetzenden natürlichen Hüftgelenks. Da zu dem Pfannenkörper 1 üblicherweise ein hier nicht dargestelltes Einsatzstück aus Kunststoff, insbesondere Polyäthylen, gehört, welches von innen in den Pfannenkörper 9 eingesetzt wird und damit in Kontakt zu der konkaven Oberfläche 28 kommt. Dieses Einsatzstück wird mit einem Schnapprand 30 in den Pfannenkörper 9 aufgenommen und durch hier nicht dargestellte Mittel verdrehgesichert gelagert. Die innere konkave Oberfläche dieses Einsatzstücks stellt die eigentliche Gleit- bzw. Reibfläche für den Kopfteil der Endoprothese dar, die am Oberschenkelknochen befestigt wird. Es ist verständlich, daß dieser Kopfteil einen gewissen Mindestdurchmesser aufweisen muß und nicht beliebig klein gestaltet werden kann. Hieraus wird verständlich, daß die exzentrische Ausbildung und Anordnung der konkaven Oberfläche 28 zu der konvexen Oberfläche 2 am Pfannenkörper 9 dazu beiträgt, die geometrischen Verhältnisse am Einsatzstück und an dem Kopfteil möglichst groß wählen zu können. Wenn der Pfannenkörper 9 dagegen selbst relativ groß gestaltet ist, kann für diese Größen, also für große Menschen, auf die exzentrische Ausbildung verzichtet werden.

Die exzentrische Ausbildung und die sich damit ergebende unterschiedliche Wandstärke hat jedoch nicht nur mit den Größenabstufungen zu tun, sondern stellt zugleich auch eine besonders beanspruchungsgerechte Ausbildung dar. Die Pfanne 1 wird in der in Figur 3 dargestellten Relativlage zur Körperachse 31 des Patienten mit seinem Kopf 32 in den Beckenknochen eingesetzt, also mit einem Winkel von etwa 50° der Ebene des Äquators 5 zur Körperachse 31. Die wesentlichen Kräfte an einem solchen mit der Pfanne 1 gebildeten Gelenk werden auch dort übertragen, wo der Pfannenkörper 9 die größte Wandstärke aufweist, während die Kräfte im Bereich der Abflachung 4 des Pols 3 bereits geringer sind und schließlich im Bereich der Kennzeichnung 12 keine nennenswerten Kräfte übertragen werden können. Dies hat mit der menschlichen Anatomie des Pfannenlagers am Beckenknochen zu tun. Bekanntlich tritt hier nach der Entknorpelung eine Knochenstruktur zutage, die eine sichel- oder mondförmige Struktur besitzt, mit abgerundeten Eckbereichen. Diese Struktur wird als Facies lunata bezeichnet. Nur in diesem Bereich liegt die Oberfläche 2 mit ihren Erhebungen 10, 14 an der knöchernen Substanz des Pfannenlagers am Beckenknochen an, weshalb einerseits die Anordnung der Erhebungen 10, 14 auf der Oberfläche auf diesen Bereich bzw. entsprechend diesem Bereich der Facies lunata beschränkt sein sollte. Im Innern der mondförmigen Struktur ist eine Vertiefung gebildet, die als Fossa bezeichnet wird. Der Eingang zu dieser Vertiefung wird als Incisura acetabuli bezeichnet. Der Pfannenkörper 9 ist nun gerade so ausgebildet und wird so eingesetzt, daß die Kennzeichnung 12 im Bereich der Incisura acetabuli zu liegen kommt, also in einem Bereich, in welchem nennenswerte Kräfte nicht übertragen werden. Das Einsetzen des Pfannenkörpers 9 erfolgt durch Einschlagen, und zwar durch Druckanwendung in Richtung der Polachse 8. Dabei greifen die scharfen Kanten 11 der Erhebungen 10 in die knöcherne Substanz ein. Sie dringen einschneidend vor, so daß der Pfannenkörper 9 bereits hier seinen ordnungsgemäßen Sitz erhält. Dabei helfen auch die zusätzlichen Erhebungen 14 mit. Diese graben sich quer zur Einschlagrichtung ein, wobei berücksichtigt werden muß, daß die knöcherne Substanz auch bis zu gewissem Grad elastisch ist. Wichtig ist, zu erkennen, daß das Einsetzen des Pfannenkörpers 9 bzw. der Pfanne 1 ohne eine Drehbewegung erfolgt, so daß verläßlich die Kennzeichnung 12 der Incisura acetabuli anliegend eingesetzt werden kann. Damit aber kommen automatisch die Durchgangslöcher 18, 19, 20 in einen solchen Bereich des Beckenknochens, wo genügend Masse zur Verfügung steht, auch vergleichsweise lange Knochenschrauben für die Sekundärverklemmung der Pfanne 1 am Beckenknochen einzusetzen, und zwar ohne daß die Gefahr besteht, daß die Knochenschrauben aus dem Beckenknochen wieder austreten und dann zu einer Verletzung des Patienten führen können. Durch die richtige Wahl der Achsen, mit denen die Durchgangslöcher 18, 19, 20 gebohrt werden und durch die richtige Ausbildung der Ansenkungen 23, 24, 25 wird ein Winkelbereich 33 (Figur 4) geschaffen, auf den das Einschrauben der Knochenschraube 21 beschränkt ist. Damit wird dem Operateur zugleich eine Hilfe gegeben, beim Bohren der Sacklöcher im Beckenknochen und dem nachfolgenden Einschrauben der Knochenschrauben 21 den richtigen Winkel zu wählen. Bei den Darstellungen der Figuren 3 und 4 wurden der Übersichtlichkeit halber die Erhebungen 10, 14 nicht dargestellt, obwohl diese natürlich vorhanden sind.

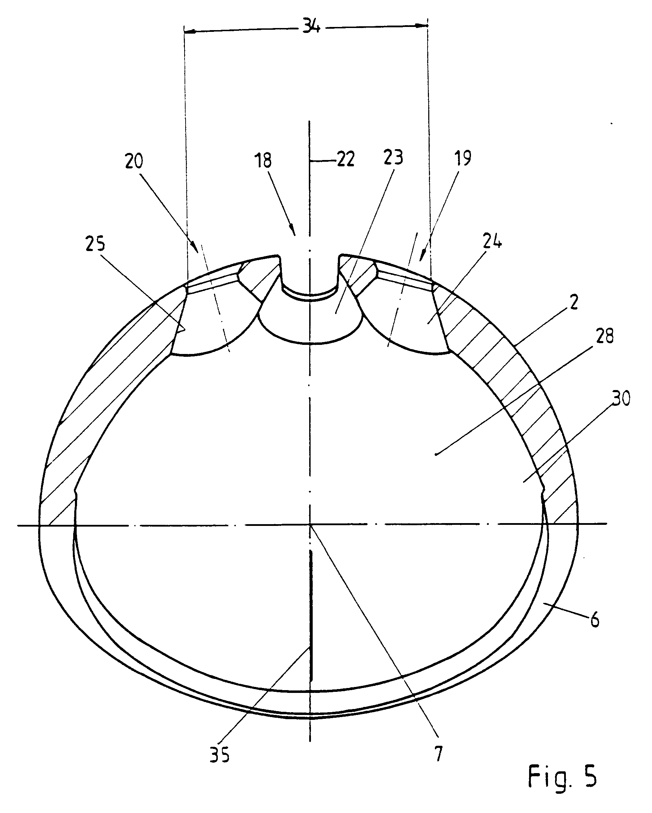

Figur 5 zeigt einen Schnitt durch eine Ebene, die einerseits durch die Linie V-V in Figur 2 und andererseits durch das Zentrum 7 festgelegt ist. Die beiden symmetrisch zur Ebene 22 angeordneten Durchgangslöcher 19 und 20 können mit ihren Ansenkungen 24 uns 25 so gebohrt und angeordnet sein, daß die Bohrachsen nicht das Zentrum 7 schneiden, sondern versetzt dazu vorgesehen sind, wie dies aus Figur 5 erkennbar ist. Die Entfernung 34 der jeweils äußeren Ränder der Durchgangslöcher 19 und 20 voneinander kann bei allen Größen der Pfannen konstant gewählt sein und beispielsweise 24 mm betragen.

Figur 6 zeigt eine weitere Ausführungsmöglichkeit der Pfanne 1. Die Erhebungen besitzen hier L-förmige Gestalt, wobei damit Erhebungen 10 mit ihren scharfen Kanten 11 mit den zusätzlichen Erhebungen 14 mit den scharfen Kanten 15 zusammengefaßt sind. Die Kanten 11 erstrecken sich in Richtung von Längengraden und die Kanten 15 in Richtung von Breitengraden. Man erkennt auch gut aus der Schnittdarstellung der linken Hälfte, wie die Höhe, also der Überstand der scharfen Kanten 11 und 15 vom Pol 3 in Richtung auf den Äquator 5 zuminnt. Dieser Überstand kann in drei Stufen von 0,8 über 1,2 bis 1,5 mm betragen. Die Länge der Schneiden 11 kann 4 bis 5 mm betragen. In Figur 6 sind weder die Kennzeichnung 12 noch die Durchgangslöcher 18, 19, 20 dargestellt, um die Zeichnung zu vereinfachen. Es ist möglich, auf die Durchgangslöcher 19, 20 zu verzichten. Ein völliger Verzicht auf eine Sekundärverklemmung mit Hilfe von Knochenschrauben 21 hätte den Nachteil, daß der feste Sitz der Pfanne 1 im Pfannenlager des Beckenknochens erst nach einem entsprechenden Zeitraum, der drei bis sechs Monate betragen kann, erreicht wird und während dieser Zeit die volle Belastung nicht möglich ist. Wird dagegen die Primärverklemmung mit Hilfe der Erhebungen 10, 14 und die Sekundärverklemmung mit Hilfe der Knochenschrauben 21 angewendet, so kann die Endoprothese sofort mit dem vollen Körpergewicht belastet werden.

Das Einsetzen einer Pfanne 1 geschieht wie folgt: Der Beckenknochen wird im Bereich des natürlichen Pfannenlagers entknorpelt, jedoch keine Knochensubstanz entfernt. Es kommt damit die Facies lunata zum Vorschein. Die Pfanne 1 wird nun so eingesetzt, daß die Kennzeichnung 12 der Incisura Acetabuli gegenüberliegt. Da dabei die Kennzeichnung 12 nicht mehr sichtbar ist und bleibt, kann - zusätzlich oder auch anstelle der Kennzeichnung 12 auf der äußeren Oberfläche 1 - auf der inneren Oberfläche 28 eine Markierung 35 (Figur 5) in Form eines Strichs o. dgl. angebracht sein. Diese Markierung 35 ist genau um 180° versetzt dem Durchgangsloch 18 gegenüber angeordnet. Die Markierung 35 verläuft auch in der Ebene 22 bzw. erstreckt sich in dieser Ebene 22. Die Markierung 35 hat nicht nur die Aufgabe, die richtige Relativlage der Pfanne 1 bei der Primärverklemmung dem Operateur zu zeigen, sondern hilft auch bei der Sekundärverklemmung. Zunächst aber wird die Pfanne 1 in dieser beschriebenen Relativlage eingeschlagen, wobei die Erhebungen 10 und 14 die Primärverklemmung herbeiführen. Damit kommen die Durchgangslöcher 18, 19, 20 in ihre richtige Relativlage zum Beckenknochen. Anschließend werden die Sacklöcher im Beckenknochen gebohrt, in die später die Knochenschrauben 21 eingesetzt werden. Das Bohren der Sacklöcher geschieht durch die Durchgangslöcher 18, 19, 20 hindurch, und zwar entweder freihändig oder mit Hilfe von Bohrbüchsen. Diese Bohrbüchsen werden in die Durchgangslöcher 18, 19, 20 eingesetzt, wobei ihre Achsen nicht das Zentrum 7 schneiden, sondern parallel bzw. in der Ebene 22 in der einen Ebene angeordnet werden. Hierbei hilft die Markierung 35 auch, weil sie die Ebene 22 andeutet. Nach dem Bohren der Sacklöcher werden die Knochenschrauben 21 eingedreht, wobei wiederum die Markierung 35 hilfreich ist. Wenn mit exzentrischen Bohrbüchsen gebohrt wurde, findet durch das Gleiten der Auflageflächen 26 an den Ansenkungen 23, 24, 25 eine geringfügige seitliche Bewegung der Pfanne 1 im Pfannenlager des Beckenknochens statt, die aber durchaus nicht nachteilig ist, sondern auch ganz bewußt herbeigeführt werden kann, um bei der Sekundärverklemmung einen noch festeren Sitz der Pfanne 1 zu erhalten. Es versteht sich, daß die Knochenschrauben 21 soweit eingedreht werden, bis ihr Kopf 27 vollständig versenkt ist. Anschließend wird das nicht dargestellte Einsatzstück aus Kunststoff eingefügt sowie die übrigen Arbeiten vollzogen.


**Patentansprüche**

1. Metallene Pfanne (1) für eine Gelenkendoprothese, die einen schalenartigen, etwa halbkugelförmigen Pfannenkörper (9) mit einer äußeren konvexen (2) und einer inneren konkaven (28) Oberfläche aufweist, wobei im halbkugeligen Pfannenkörper mindestens ein Durchgangsloch (18) mit Ansenkung zwischen Pol (3) und Äquator (5) für den versenkten Einsatz einer Knochenschraube (21) vorgesehen ist, dadurch gekennzeichnet, daß der Pfannenkörper (9) auf seiner äußeren konvexen Oberfläche (2) entsprechend der Form der Facies lunata angeordnete Erhebungen (10, 14) mit scharfen Kanten (11, 15) aufweist, daß auf der äußeren Oberfläche (2) eine feldmäßige Kennzeichnung (12) entsprechend der Lage der Incisura acetabuli und entsprechend auf der inneren Oberfläche (28) eine nach Art eines Längengrads verlaufende Markierung (35) vorgesehen sind, und daß das Durchgangsloch (18) auf der um 180° versetzt gegenüberliegenden Seite der Kennzeichnung (12) und der Markierung (35) angeordnet ist.

2. Pfanne nach Anspruch 1, dadurch gekennzeichnet, daß die Erhebungen (10, 14) mit ihren scharfen Kanten (11, 15) im wesentlichen in Richtung des Verlaufs von Längen- und Breitengraden an der Kugel angeordnet sind.

3. Pfanne nach Anspruch 2, dadurch gekennzeichnet, daß die Erhebungen (10, 14) zumindest teilweise U-förmige Gestalt aufweisen, wobei die Öffnung des U dem Pol (3) der Pfanne (1) zugekehrt angeordnet ist.

4. Pfanne nach Anspruch 1, dadurch gekennzeichnet, daß das Durchgangsloch (18) als Langloch ausgebildet und in Richtung des Verlaufs des Längengrads angeordnet ist.

5. Pfanne nach Anspruch 4, dadurch gekennzeichnet, daß zusätzlich zu dem als Langloch ausgebildeten Durchgangsloch (18) zwei weitere Durchgangslöcher (19, 20) in symmetrischer Anordnung zu der von dem Langloch aufgespannten Ebene (22) vorgesehen sind.

6. Pfanne nach Anspruch 1 bis 5, dadurch gekennzeichnet, daß die innere Oberfläche (28) des Pfannenkörpers (9) gegenüber der äußeren Oberfläche (2) des Pfannenkörpers (9) exzentrisch angeordnet ist und die Lage der Exzentrizität so gewählt ist, daß im Bereich der Durchgangslöcher (18, 19, 20) eine große und im Bereich der Incisura acetabuli eine geringe Wandstärke entsteht.

7. Pfanne nach Anspruch 1, dadurch gekennzeichnet, daß der halbkugelförmige Pfannenkörper (9) im Bereich seines Pols (3) gegenüber der Kreis- bzw. Kugelform mit einer Abflachung (4) versehen ist.

**Claims**

1. Metal cup (1) for a joint endoprosthesis, which comprises a shell-shaped and approximately semispherical cup element (9) with an outer convex surface (2) and an inner concave surface (28), there being provided in the semispherical cup element at least one through-hole (18) with countersink, between pole (3) and equator (5), for the countersunk introduction of a bone screw (21), characterised in that the cup element (9) has, on its outer convex surface (2), raised portions (10, 14) with sharp edges (11, 15) arranged corresponding to the form of the lunate surface, in that an area identification (12) is provided on the outer surface (2) corresponding to the position of the acetabular notch and a marking (35) extending in the manner of a degree of longitude is provided correspondingly on the inner surface (28), and in that the through-hole (18) is arranged on the side lying offset by 180° opposite the identification (12) and the marking (35).

2. Cup according to Claim 1, characterised in that the raised portions (10, 14) with their sharp edges (11, 15) are arranged essentially in the direction of the course of degrees of longitude and latitude on the sphere.

3. Cup according to Claim 2, characterised in that the raised portions (10, 14) have an at least partially U-shaped configuration, the opening of the U being arranged facing towards the pole (3) of the cup (1).

4. Cup according to Claim 1, characterised in that the through-hole (18) is designed as an oblong hole and is arranged in the direction of the course of the degree of longitude.

5. Cup according to Claim 4, characterised in that, in addition to the through-hole (18) designed as an oblong hole, two further through-holes (19, 20) are provided in symmetrical arrangement relative to the plane (22) set by the oblong hole.

6. Cup according to Claim 1 to 5, characterised in that the inner surface (28) of the cup element (9) is arranged eccentric relative to the outer surface (2) of the cup element (9), and the position of the eccentricity is chosen such that there is a large wall thickness in the area of the through-holes (18, 19, 20) and a small wall thickness in the area of the acetabular notch.

7. Cup according to Claim 1, characterised in that the semispherical cup element (9) is provided with a flattened part (4) in the area of its pole (3) with respect to the circular or spherical shape.

**Revendications**

1. Cuvette métallique (1) pour une endoprothèse d'articulation, présentant un corps de cuvette (9) sensiblement hémisphérique avec une surface externe convexe (2) et une surface interne concave (28), tandis qu'il est prévu dans le corps de cuvette hémisphérique (9) au moins un trou de passage (18) chanfreiné, entre le pôle (3) et l'équateur (5) pour l'insertion d'une vis à os (21) à tête noyée, caractérisée en ce que le corps de cuvette (9) présente sur sa surface externe convexe (2) des protubérances (10, 14) avec des bords aigus (11, 15) disposées de manière correspondant à la forme de la facies lunata, en ce que sont prévus sur la surface externe (2) un repère topographique (12) correspondant à la position de l'incisura acetabuli, et sur la surface interne (28) un marquage (35) s'étendant à la manière d'un degré de longitude, et en ce que le trou de passage (18) est disposé sur le côté opposé, décalé de 180°, par rapport au repère (12) et au marquage (35).

2. Cuvette selon la revendication 1, caractérisée en ce que les protubérances (10, 14) avec leurs bords aigus (11, 15) sont disposées essentiellement dans le sens où des degrés de longitude et des degrés de latitude s'étendent sur une sphère.

3. Cuvette selon la revendication 2, caractérisée en ce que les protubérances (10, 14) présentent une conformation au moins partiellement en forme de U, l'ouverture de l'U étant tournée vers le pôle (3) de la cuvette (1).

4. Cuvette selon la revendication 1, caractérisée en ce que le trou de passage (18) est sous la forme d'un trou allongé et est disposé dans le sens d'extension du degré de longitude.

5. Cuvette selon la revendication 4, caractérisée en ce qu'en plus du trou de passage (18) sous la forme d'un trou allongé sont prévus deux autres trous de passage (19, 20) disposés symétriquement par rapport au plan (22) fixé par le trou allongé.

6. Cuvette selon les revendications 1 à 5, caractérisée en ce que la surface interne (28) du corps de cuvette (9) est excentrée par rapport à la surface externe (2) du corps de cuvette (9) et la position de l'excentricité est choisie de telle sorte qu'il en résulte une grande épaisseur de paroi dans la région des trous de passage (18, 19, 20) et une faible épaisseur de paroi dans la région de l'incisura acetabuli.

7. Cuvette selon la revendication 1, caractérisée en ce que le corps de cuvette hémisphérique (9) est muni, dans la région de son pôle (3) d'un aplatissement (4) par rapport à la forme circulaire ou sphérique.

Fig. 1

Fig. 2

EP 0 380 045 B1

Fig. 3

Fig. 4

EP 0 380 045 B1

Fig. 5

Fig. 6